Europäisches Patentamt

European Patent Office

Office européen des brevets

(19)

(11) Publication number: **0 118 964**
**B1**

(12) **EUROPEAN PATENT SPECIFICATION**

(45) Date of publication of patent specification: **19.08.87**

(51) Int. Cl.⁴: **G 01 N 33/14,** G 01 N 7/14

(21) Application number: **84200347.7**

(22) Date of filing: **09.03.84**

(54) **Apparatus for measuring the gas concentration of a gas-containing liquid.**

(30) Priority: **10.03.83 NL 8300881**

(43) Date of publication of application:
**19.09.84 Bulletin 84/38**

(45) Publication of the grant of the patent:
**19.08.87 Bulletin 87/34**

(84) Designated Contracting States:
**BE DE FR GB NL**

(56) References cited:
**DE-A-1 448 162**
**FR-A-2 110 826**
**GB-A-1 470 275**
**GB-A-2 061 500**
**US-A-3 077 765**
**US-A-4 276 769**
**US-A-4 299 794**

(73) Proprietor: **Haffmans B.V.**
**Kaldenkerkerweg 36**
**NL-5913 AG Venlo (NL)**

(72) Inventor: **Haffmans, Hubertus Ernest F.M.**
**Koninginnesingel 58**
**NL-5911 KE Venlo (NL)**

(74) Representative: **Urbanus, Henricus Maria, Ir.**
**et al**
**c/o Vereenigde Octrooibureaux Nieuwe**
**Parklaan 107**
**NL-2587 BP 's-Gravenhage (NL)**

Courier Press, Leamington Spa, England.

# Description

The invention relates to an apparatus for measuring the gas concentration of a gas-containing liquid, comprising a first chamber adapted to be connected to a pressure gauge and having a supply opening through which said chamber can be filled with the liquid to be analysed.

Although the apparatus according to the invention can be used for measuring the gas concentration of gas-containing liquids, the invention is directed in particular to the measurement of the $CO_2$ content of beer. The invention will therefore be described hereinafter for measuring the $CO_2$ content of beer, without being restricted thereto.

In the manufacture of beer it is highly important for the $CO_2$ content of the beer to be kept at the proper value. In case the $CO_2$ content is too low, the refreshing effect of the beer is lost, while an excessive $CO_2$ content is undesirable as well. Various apparatuses for measuring the $CO_2$ content of beer are already known, and the operation of these prior art apparatuses is based on Henry's Law, which is worded as follows: "At a constant temperature, the mass of a gas dissolved in a given solvent volume, is directly proportional to the partial pressure of this gas surmounting the liquid".

Henry's Law applies only to a closed system and if there is an equilibrium between the gas in the liquid and the gas surmounting the liquid. Henry's equation can be written in symbols as follows:

$$\frac{m}{V}=f.p \ (T=\text{constant})$$

wherein
m=mass of the gas
V=volume of the solvent
f=Henry's constant
p=pressure of the gas surmounting the liquid
T=temperature in °K.

$$\text{The gas concentration}=c=\frac{m}{V}.$$

DE—A—1448162 describes a system for measuring the $CO_2$ content of beer, in which the main conduit for the beer accommodates a branch wherein there is positioned a pump which transports a quantity of beer to be analysed from the main conduit to a chamber. It has been found in practice that the pump constitutes a source of disturbances, while a further drawback is that the pump consumes energy.

Therefore, it is an object of the invention to provide an apparatus for measuring the $CO_2$ content of beer, which has a simple construction, can be installed in an existing beer line in a simple manner, consumes a minimum quantity of energy and is very reliable in operation.

To this end, the invention provides an apparatus of the above described type wherein the first chamber contains a first piston provided at the top with a recess in the body of the piston, and being in contact with the sidewalls of the first chamber, and a second piston in the recess in the body of the first piston which is in contact with the sidewall of said recess, a resilient member being provided between the bottom of the recess and the bottom of the second piston, there being arranged means for moving the first piston in the first chamber, and means for moving the second piston relatively to the first piston, against the forces of the resilient member.

As will be explained in more detail in the following, after the first chamber has been filled with beer and the supply opening has been closed, by movement of the second piston in said first chamber, a space can be formed through which gas from the liquid can escape, which space will be referred to in the following as the gas volume. After the gas volume has been formed, it is necessary for the determination of the $CO_2$ content of the beer by means of Henry's Law, that an equilibrium is effected between the gas in the liquid and the gas in the space surmounting the liquid. When this equilibrium exists, the $CO_2$ concentration can be determined by measuring the equilibrium pressure and by measuring the temperature of the beer. Since the equilibrium in aqueous liquids, such a beer, is established only very slowly, it is desirable to accelerate the formation of this equilibrium in the first place so that one may be sure that full equilibrium has actually been brought about and in the second place so that the measurement may not be too time-consuming.

DE—A—1448162 already proposes to accelerate the formation of the equilibrium between the gas in the beer and the gas in the surmounting gas volume by installing a pair of electrodes in the beer to be analysed, with a potential difference being applied therebetween. Due to the occurring electrolysis, gas bubbles are formed on the electrodes that rise in the beer and which form nuclei for the $CO_2$ gas separating from the beer. It has been found that an equilibrium between the gas in the beer and the gas in the gas volume can thus be quickly realised.

When the equilibrium has been effected, the $CO_2$ content of the beer can be calculated e.g. by using the apparatus and the method described in Dutch patent application 7701200.

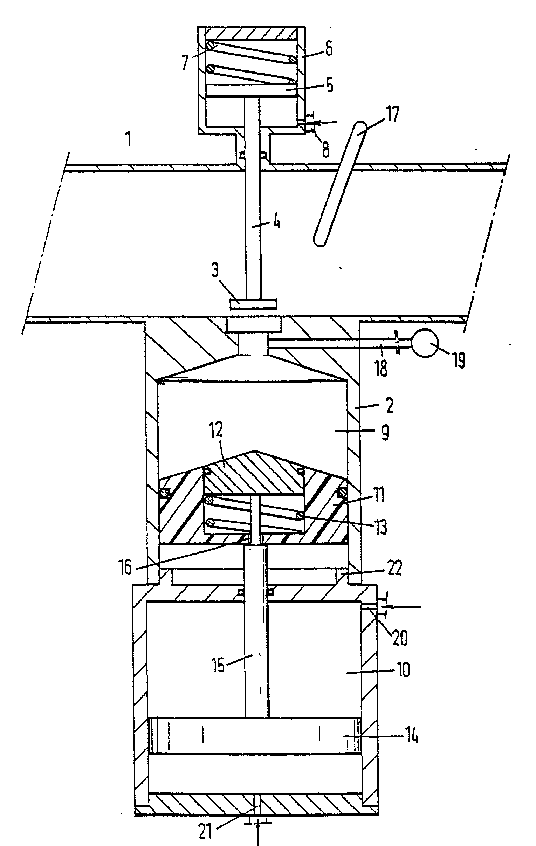

One embodiment of the apparatus according to the invention will now be described, by way of example, with reference to the accompanying drawing wherein the figure is a cross-sectional view of the apparatus. The figure shows a conduit 1 traversed by the beer to be analysed. To the sidewall of conduit 1 there is attached a hollow measuring cylinder 2 communicating with the conduit 1. The connection between the measuring cylinder 2 and the conduit 1 can be shut off by a valve 3 mounted on one end of a piston rod 4. To the other end of said piston rod there is attached in a chamber 6 a piston 5 adapted for movement in chamber 6. Chamber 6 is disposed on the wall of conduit 1 opposite the measuring cylinder. Between the rear wall of chamber 6 and piston 5 there is provided a compression spring 7 which, in rest, causes the valve 3 to shut off the connection between conduit 1 and the interior of measuring cylinder 2. An opening for supplying pressurized medium is arranged in the sidewall of chamber 6 between the front wall of the chamber disposed adjacent conduit 1 and the front of piston 5, under the influence of which the piston 5 can be moved against the force of spring 7 for clearing the connection between conduit 1 and measuring cylinder 2.

The hollow measuring cylinder 2 comprises a first chamber 9 and a second chamber 10, which is essentially co-extensive with the first chamber. Chambers 9 and 10 are entirely separated from one another by a partition. Chamber 9 accommodates a piston 11 contacting the wall of said chamber. The top of piston 11 comprises a recessed central portion wherein a second piston 12 is placed. Between the bottom of piston 12 and the bottom of the recessed central portion in piston 11, there is arranged a compression spring 13 pressing piston 12 from the central portion upwardly.

The second chamber 10 of the measuring cylinder accommodates a piston 14 connected to piston 12 by means of a piston rod 15. Piston rod 15 is thinner at the end connected to piston 12 than along the major part of the length thereof, so that adjacent piston 11, at the location where the piston rod 15 is passed through an opening 16 in piston 11, there is formed a stop which abuts against the lower surface of piston 11, thus preventing piston 12 from being entirely pressed out of the central portion of piston 11 under the influence of spring 13. At the rear of chamber 9, there is formed a stop against which piston 11 abuts in its lower position. Piston 14 has then not yet reached its lowermost position and when piston 14 is moved further downwards, in a manner described hereinafter, piston 12 will move downwardly in the recess in piston 11 against the force of spring 13.

A temperature gauge 17 is positioned in conduit 1, while adjacent the connection between conduit 1 and chamber 9 there is provided a conduit 18 connected to a pressure gauge 19. Finally, adjacent the front wall of chamber 10 there is arranged a supply opening 20 for a fluid under pressure and adjacent the rear wall of chamber 10, under piston 14, there is also provided a supply opening 21 for a fluid under pressure. As will be further described in the following, the piston 11 and the piston rod 15 are made preferably of an electrically insulating material.

The circumferential surfaces of the various pistons, just as the passageways for the piston rods, are fitted with suitable, known per se sealing means, ensuring a proper seal between the various spaces and a proper operation of the pistons.

The operation of the apparatus is as follows.

The starting point is the situation in which chamber 9 is filled with beer of a preceding measurement, with piston 11 abutting against stop 22 and piston 12 being in its lowermost position relative to piston 11, into which position piston 12 has been brought, against the force of spring 13, by piston 14, which is also in its lowermost position. Valve 3 shuts off the connection between conduit 1 and chamber 9.

Subsequently, a new sample to be analysed is taken from the beer in conduit 1 by pressurizing opening 8 so that piston 5 is moved against the force of spring 7, and valve 3 clears the connection between conduit 1 and chamber 9. Also opening 21 is pressurized, so that piston 14 moves to the front wall of chamber 10, when first piston 12 is brought to its uppermost position relative to piston 11 and subsequently, when the thickened portion of piston rod 15 abuts against the bottom of piston 11, the combination of the pistons 11 and 12 is moved to the front wall of chamber 9, with the preceding beer sample from chamber 9 being returned to conduit 1.

After the combination of pistons 11 and 12 has reached its uppermost position, the supply opening 21 is cleared. By the pressure of the beer in conduit 1, the piston combination 11, 12 is again moved backwards in chamber 9, with beer flowing into chamber 9. The spring 13 is so strong that piston 12 does not move relatively to piston 11 under the influence of the beer flowing into chamber 9. When piston 11 abuts against stop 22, the entire chamber 9 is filled with beer and the supply opening 8 is cleared with valve 3, under the influence of spring 7, shutting off the connection between chamber 9 and conduit 1.

Subsequently, a gas volume is formed in chamber 9 by pressurizing supply opening 20. This pressure is so high that the force of spring 13 is overcome by means of piston 14 and piston rod 15, thereby moving piston 12 downwardly in the recess in piston 11. When piston 14 abuts against the rear wall of chamber 10, piston 12 cannot be moved further and a space is formed in chamber 9 in a simple manner to which the $CO_2$ present in the beer can escape.

As described in the above, piston 11 and piston rod 15 are made preferably of an electrically insulating material. This makes it possible to apply a potential difference between the piston 12 and the wall of chamber 9, both made of an electrically conductive material. Chamber 9 is normally connected to the ground potential. By applying the potential difference, there is rapidly produced, under the influence of electrolysis, a gas equilibrium between the $CO_2$ surmounting the beer and the $CO_2$ in the beer.

Finally, the pressure of the gas surmounting the beer can be measured with the pressure gauge 19 and the temperature of the beer with thermometer 17, with the temperature difference between the beer sample in chamber 9 and the beer in conduit 1 being zero or negligibly small. When the relation between Henry's constant and the temperature for the specific system is known, it is possible to determine the $CO_2$ content from the values measured, e.g. by using the apparatus and the process described in Dutch patent application 7701200 or e.g. by means of a process computer.

It will be clear that the apparatus according to the invention offers a simple, reliable and relatively inexpensive possibility of determining the gas concentration of a gas-containing liquid, in particular the $CO_2$ concentration of beer, said apparatus being readily adaptable to existing beer conduits. A great many modifications obvious to those skilled in the art are possible within the scope of the invention as defined in the claims.

## Claims

1. An apparatus for measuring the gas concentration of a gas-containing liquid, comprising a first chamber (9) adapted to be connected to a pressure gauge, and having a supply opening through which said first chamber (9) can be filled with the liquid to be analysed, characterized in that the first chamber (9) is fitted with a first piston (11) having at the top a recess in the body of the piston (11) and contacting the sidewalls of the first chamber (9), and with a second piston (12) in the recess in the body of the first piston (11) contacting the sidewall of said recess, while between the bottom of the recess and the bottom of the second piston (12) there is provided a first resilient member (13), and there are provided means (14, 15) for moving the first piston (11) in the first chamber (9) and means (14, 15) for moving the second piston (12) relatively to the first piston (11), against the force of the resilient member (13).

2. An apparatus according to claim 1, characterized in that the means (14, 15) for moving the first piston (11) and for moving the second piston (12) relatively to the first piston (11) comprise a third piston (14) arranged in a second chamber (10) essentially co-extensive with the first chamber (9), the third piston (14) being connected to the second piston (12) by means of a first piston rod (15), which first piston rod (15) is passed through an opening (16) in the bottom of the recess in the first piston (11) and comprises a thickened portion which, in the rest position of the piston rod (15), abuts against the underside of said bottom.

3. An apparatus according to claim 1 or 2, with the apparatus being connected to a conduit (1) through which the gas-containing liquid to be analysed flows, with the supply opening being formed in the wall of said conduit (1), characterized in that there is provided a valve (3) for opening and shutting off the supply opening, said valve (3) being connected by means of a second piston rod (4) to a fourth piston (5) in a third chamber (6), which third chamber (6) is connected to the conduit (1) at a location opposite the supply opening, in that between the fourth piston (5) and the rear wall of the third chamber (6) there is provided a second resilient member (7), and in that, in the space situated at the side of the fourth piston (5) remote from the second resilient member (7), the wall of the third chamber (6) contains an opening (8) for supplying a fluid under pressure.

4. An apparatus according to claim 2 or 3, characterized in that the wall of the second chamber (10) in the space above the third piston (14) and in the space underneath the third piston (14) contains openings (20, 21) for supplying a fluid under pressure.

5. An apparatus according to any one of claims 1—4, characterized in that a means (19) for measuring gas pressure is connected to the first chamber (9) adjacent the supply opening and that means (17) are provided for measuring the temperature of the liquid to be analysed.

6. An apparatus according to at least any one of the preceding claims, characterized in that the first piston (11) and the first piston rod (15) connected to the second piston (12) are made of an electrically insulating material and means are provided for bringing the second piston (12) at an electric potential relative to the wall of the first chamber (9).

## Patentansprüche

1. Vorrichtung zur Messung der Gaskonzentration einer Gas enthaltenden Flüssigkeit mit einer ersten Kammer, die mit einem Druckmeßgerät verbindbar ist und die eine Zuführöffnung besitzt, durch welche die erste Kammer (9) mit der zu analysierenden Flüssigkeit gefüllt werden kann, dadurch gekennzeichnet, daß in die erste Kammer (9) ein erster Kolben (11) eingebaut ist, welcher an der Oberseite eine Ausnehmung in dem Kolbenkörper (11) besitzt und die Seitenwände der ersten Kammer (9) berührt, und daß ein zweiter Kolben (12) in die Ausnehmung des Körpers des ersten Kolbens (11) eingebaut ist, der die Seitenwände der Ausnehmung berührt, wobei zwischen dem Boden der Ausnehmung und dem Boden des zweiten Kolbens (12) ein erstes Federelement (13) vorgesehen ist, und daß Einrichtungen (14, 15) zur Bewegung des ersten Kolbens (14) in der ersten Kammer (9) und Einrichtungen (14, 15) zur Bewegung des zweiten Kolbens (12) relativ zu dem ersten Kolben (11) gegen die Kraft des Filterelements (13) vorgesehen sind.

2. Vorrichtung nach Anspruch 1, dadurch gekennzeichnet, daß die Einrichtungen (14, 15) zur Bewegung des ersten Kolbens (11) und zur Bewegung des zweiten Kolbens (12) relativ zu dem ersten Kolben (11) einen dritten Kolben (14) aufweist, der in einer zweiten Kammer (10) angeordnet ist, die sich zusammen mit der ersten Kammer (9) erstreckt, wobei der dritte Kolben (14) mit dem zweiten Kolben (12) mittels einer ersten Kolbenstange (15) verbunden ist, wobei diese erste Kolbenstange (15) durch eine Öffnung (16) in dem Boden der Ausnehmung in dem ersten Kolben (11) geführt ist, und einen vergrößerten Abschnitt aufweist, der in der Ruhestellung der Kolbenstange (15) an der Unterseite des Bodens anstößt.

3. Vorrichtung nach Anspruch 1 oder 2, bei der die Vorrichtung mit einer Leitung (1) verbunden ist, durch welche die zu analysierende Gas enthaltende Flüssigkeit strömt, wobei die Zuführöffnung in der Wand der Leitung (1) gebildet ist, dadurch gekennzeichnet, daß ein Ventil (3) zum Öffnen und Schließen der Zuführöffnung vorgesehen ist, wobei das Ventil (3) mittels einer zweiten Kolbenstange (4) mit einem vierten Kolben (5) in einer dritten Kammer (6) verbunden ist, wobei diese dritte Kammer (6) mit der Leitung (1) an einer Stelle gegenüber der Zuführöffnung verbunden ist, daß zwischen dem vierten Kolben (5) und der Rückwand der dritten Kammer (6) ein zweites Federelement (7) vorgesehen ist, und daß in dem Raum, der an der Seite des vierten Kolbens (5) entfernt von dem zweiten Federlerment (7) liegt, die Wand der dritten Kammer (6) eine Öffnung (8) für das Zuführen eines Fluids unter Druck aufweist.

4. Vorrichtung nach Anspruch 2 oder 3, dadurch gekennzeichnet, daß die Wand der zweiten Kammer (10) in dem Raum oberhalb des dritten Kolbens (14) und in dem Raum unterhalb des dritten Kolbens (14) Öffnungen (20, 21) für das Zuführen von Flüssigkeit unter Druck aufweist.

5. Vorrichtung nach einem der Ansprüche 1—4, dadurch gekennzeichnet, daß eine Einrichtung (19) zur Messung von Gasdruck mit der ersten Kammer (9) nahe der Zuführöffnung verbunden ist und daß Einrichtungen (17) zur Messung der Temperatur der zu analysierenden Flüssigkeit vorgesehen sind.

6. Vorrichtung nach wenigstens einem der vorangehenden Ansprüche, dadurch gekennzeichnet, daß der erste Kolben (11) und die erste Kolbenstange (15), die mit dem zweiten Kolben (12) verbunden ist, aus einem elektrisch isolierenden Material hergestellt sind, und daß Einrichtungen vorgesehen sind, um den zweiten Kolben (12) auf ein elektrisches Potential relativ zu der Wand der ersten Kammer (9) zu bringen.

**Revendications**

1. Un appareil de mesure de la concentration en gaz d'un liquide contenant du gaz, comportant une première chambre (9) destinée à être reliée à un appareil de mesure de pression et comportant une ouverture d'alimentation à travers laquelle ladite première chambre (9) peut être remplie du liquide à analyser, caractérisé en ce que la première chambre (9) est équipée d'un premier piston (11) présentant au sommet une cavité dans le corps du piston (11) et venant en contact avec les parois latérales de la première chambre (9) et avec un second piston (12) dans la cavité du corps du premier piston (11) venant en contact avec la paroi latérale de ladite cavité, tandis qu'entre le fond de la cavité et le fond du second piston (12), est prévu un premier organe élastique (13), et des moyens (14, 15) pour déplacer le premier piston (11) dans la première chambre (9) et des moyens (14, 15) pour déplacer le second piston (12) par rapport au premier piston (11) à l'encontre de la force exercée par l'organe élastique (13).

2. Un appareil selon la revendication 1, caractérisé en ce que les moyens (14, 15) pour déplacer le premier piston (11) et pour déplacer le second piston (12) par rapport au premier piston (11) comporte un troisième piston (14) agencé dans une seconde chambre (10) qui est sensiblement jointive à la première chambre (9), le troisième piston (14) étant relié au second piston (12) à l'aide d'une première tige de piston (15) qui traverse une ouverture (16) dans le fond de la cavité du premier piston (11) et qui comporte une partie plus épaisse qui vient en butée, en position de repos de la tige de piston (15), sur la face inférieure dudit fond.

3. Un appareil selon la revendication 1 ou 2, l'appareil étant relié à une conduite (1) à travers laquelle s'écoule le liquide contenant du gaz à analyser, l'ouverture d'alimentation étant formée dans la paroi dudit conduit (1), caractérisé en ce qu'il est prévu une valve (3) pour ouvrir et fermer l'ouverture d'alimentation, ladite valve (3) étant reliée à l'aide d'une seconde tige de piston (4) à un quatrième piston (5) dans une troisième chambre (6), laquelle troisième chambre (6) est reliée à la conduite (1) en un endroit situé à l'opposé de l'ouverture d'alimentation, en ce que, entre le quatrième piston (5) et la paroi arrière de la troisième chambre (6), est prévu un second organe élastique (7) et en ce que, dans l'espace situé sur le côté du quatrième piston (5) à distance du second organe élastique (7), la paroi de la troisième chambre (6) est munie une ouverture (8) d'amenée d'un fluide sous pression.

4. Un appareil selon la revendication 2 ou 3, caractérisé en ce que la paroi de la seconde chambre (10) dans l'espace situé au-dessus du troisième poiston (14) et dans l'espace situé endessous du troisième piston (14) comporte des ouvertures (20, 21) aptes à fournir un fluide sous pression.

5. Un appareil selon l'une quelconque des revendications 1 à 4, caractérisé en ce qu'un moyen (19) de mesure de la pression de gaz est relié à la première chambre (9) adjacente à l'ouverture d'alimentation et en ce que des moyens (17) sont prévus pour mesurer la température du liquide à analyser.

6. Un appareil selon au moins l'une quelconque des revendications précédentes, caractérisé en ce que le premier piston (11) et la première tige de piston (15) reliée au second piston (12) sont réalisés en un matériau électriquement isolant et en ce que des moyens sont prévus pour amener le second piston (12) à un potentiel électrique par rapport à la paroi de la première chambre (9).